# EUROPEAN PATENT SPECIFICATION

(11) **EP 1 289 534 B1**
(45) Date of publication and mention of the grant of the patent: **30.11.2005**
(21) Application number: 01935896.9
(22) Date of filing: 25.05.2001
(51) Int. Cl.: A61K 35/12, A61K 35/14, A61P 29/00, A61P 17/00, A61P 19/02, A61P 37/00, A61P 3/10, A61P 9/10, A61P 25/28

(54) **APOPTOTIC ENTITIES FOR USE IN TREATMENT OF T-CELL-MEDIATED AND INFLAMMATORY DISORDERS**
APOPTOTISCHE KÖRPER ZUR VERWENDUNG IN DER BEHANDLUNG VON T-ZELL-VERMITTELTEN- UND ENTZÜNDUNGSERKRANKUNGEN
ENTITES APOPTOTIQUES DESTINEES A ETRE UTILISEES POUR LE TRAITEMENT DE TROUBLES INFLAMMATOIRES LIES AUX LYMPHOCYTES T

(30) Priority: 25.05.2000 CA 2309518
(43) Date of publication of application: 12.03.2003
(73) Proprietor: Vasogen Ireland Limited, Shannon, County Clare (IE)
(72) Inventor: SAUDER, Daniel, Toronto, Ontario M2P 1L4 (CA); MANDEL, Arkady, North York, Ontario M2R 3S7 (CA); BOLTON, Anthony E., Bakewell, Derbyshire DE45 1BJ (GB)
(74) Representative: Gowshall, Jonathan Vallance
(86) International application number: PCT/CA2001/000758
(87) International publication number: WO 2001/089536

(56) References cited:
- WO-A-98/07436
- G.M. SHIVJI ET AL.: "EFFECTS OF VAS972 THERAPY ON ELLERGIC CONTACT HYPERSENSITIVITY" JOURNAL OF INVESTIGATIVE DERMATOLOGY, NEW YORK, NY, US, vol. 114, no. 4, April 2000 (2000-04), page 862 XP000960563 ISSN: 0022-202X
- B. BODEY ET AL.: "APOPTOSIS IN THE MAMMALIAN THYMUS DURING NORMAL HISTOGENESIS AND UNDER VARIOUS IN VITRO AND IN VIVO EXPERIMENTAL CONDITIONS." IN VIVO, vol. 12, no. 1, 1998, pages 123-134, XP001064255 Athens, GR
- G.M. SHIVJI ET AL.: "THE EFFECT OF VAS972 ON ALLERGIC CONTACT HYPERSENSITIVITY" JOURNAL OF CUTANEOUS MEDICINE AND SURGERY, DECKER PERIODICALS FOR THE CANADIAN DERMATOLOGY ASSOCIATION,, CA, vol. 4, no. 3, July 2000 (2000-07), pages 132-137, XP000960566 ISSN: 1203-4754

## Description

### Field of the Invention

This invention relates to biochemical and biological compositions which can be used in the treatment and/or prophylaxis of various T-cell-mediated and inflammatory medical disorders in mammalian patients. More particularly, treatment and prophylaxis of T-cell-mediated (e.g. autoimmune) and inflammatory medical disorders can be carried out by administration of compositions containing mammalian cellular materials and fragments thereof.

### Background of the Invention

Two mechanisms of cell death in the body are recognized, necrosis and apoptosis. Apoptosis is the process of programmed cell death, described by Kerr et al. in 1992 [Kerr JFR, Wyllie AH, Currie AR (1992). "Apoptosis: a basic biological phenomenon with wide-ranging implications in tissue kinetics," *British Journal* of *Cancer* **26:** 239-257], by which steady-state levels of the various organ systems and tissues in the body are maintained as continuous cell division and differentiation takes place. Cells undergoing apoptosis often exhibit distinctive morphological changes such as a pronounced decrease in cell volume, modification of the cytoskeletons resulting in pronounced membrane blebbing, a condensation of the chromatin, and degradation of the DNA into oligonucleosomal fragments.

Following these morphological changes, an apoptotic cell may break-up into a number of small fragments known as apoptotic bodies, comprising membrane-bound bodies containing intact organelles, chromatin, etc. Apoptotic bodies are normally rapidly removed from the body by phagocytosis, by macrophages, dendritic cells and other antigen-presenting cells, before they can become lysed and release their potentially pro-inflammatory intracellular contents.

In simple outline, apoptosis is thought to proceed as follows. Three phases can be identified in the apoptotic mechanism of programmed cell death:
Induction phase;
Effector phase;and
Degradation phase.

The induction phase is dependent, in part, on specific interactions of death-inducing signals at the cell surface membrane. One common signal is initiated by the binding of specific ligands to receptors of the TNF receptor family present on the cell membrane. One important such receptor is Fas (APO-1, CD95), which interacts with Fas-ligand to initiate apoptosis.

The effector phase, activated by the binding of receptors and ligands of the induction phase, leads to the activation of caspases, cystinyl-aspartate-requiring proteinases (proteolytic enzymes), including caspases 1 and 8. This activation may be associated with a change in the permeability of mitochondria, allowing the release of cytochrome-c which is involved in caspase activation. Activated caspases initiate a chain of lethal proteolytic events culminating in the changes in chromatin and cytoskeletal components seen in apoptosis.

Many cells undergoing apoptosis can be identified by a characteristic 'laddering' of DNA seen on agarose gel electrophoresis, resulting from cleavage of DNA into a series of fragments. These changes occur a few hours before death of the cell as defined by the ability of a cell to exclude vital dyes. The appearance of DNA laddering on agarose gel electrophoresis following extraction of DNA from cells is one recognised method of identification of apoptosis in cells [Loo, D.T. and Rillema, J.R. (1998) "Measurement of Cell Death," *Methods in Cell Biology* **57:** 251-264], although it is not always sensitive enough to detect apoptosis. *In situ* labelling of nuclear DNA fragmentation, for example, using commercially available terminal dUTP nick end labelling (TUNEL) assays, are an alternative and more reproducible measure for the determination of fragmented DNA in apoptotic cells and cells undergoing apoptosis [Gavrieli Y, Sherman Y, Ben-Sasson SA (1992) "Identification of programmed cell death in situ via specific labelling of nuclear DNA fragmentation," *Journal of Cell Biology* **119:** 493-501].

During apoptosis, phosphatidylserine becomes exposed externally on the cell membrane [Fadok VA, Voelker DR, Campbell PA, Cohen JJ, Bratton DL, Henson PM (1992), "Exposure of phosphatidylserine on the surface of apoptotic lymphocytes triggers specific recognition and removal by macrophages". *Journal of Immunology* **148:** 2207-2216] and this exposed phosphatidylserine binds to specific receptors to mediate the uptake and clearance of apoptotic cells in mammals [Fadok VA, Bratton DL, Rose DM, Pearson A, Ezekewitz RAB, Henson PM (2000) "A receptor for phosphatidylserine-specific clearance of apoptotic cells," *Nature* **405**: 85-90]. The surface expression of phosphatidylserine on cells is another recognized method of identification of apoptotic cells.

Changes in mitochondrial integrity are intimately associated with apoptosis, resulting in alterations in mitochondrial membrane permeability and the release of cytochrome-c from the mitochondria into the cell cytoplasm [Susin, S.A., Lorenzo, H.K., Zamzami, N., Marzo, I, Brenner, C., Larochette, N., Prevost, M.C., Alzari, P.M. and Kroemer, G. (1999) "Mitochondrial Release of Caspase-2 and -9 during the Apoptotic Process", *Journal of Experimental Medicine,* **189:** 381 - 394]. Measurement of changes in mitochondrial membrane potential, reflecting changes in mitochondrial membrane permeability, is another recognized method of identification of apoptotic cells.

A number of other methods of identification of cells undergoing apoptosis and of apoptotic cells, many using monoclonal antibodies against specific markers for apoptotic cells, have also been described in the scientific literature.

Methods of quantifying apoptotic cells and apoptotic bodies in a cellular composition are known and readily practiced by persons of skill in the art. Techniques include staining of the treated cell population, with an appropriate, selective dye such as fluorescein-conjugated annexin V, followed by incubation and analysis by flow cytometry.

Necrosis, in contrast, is cell death of a pathological nature, resulting from injury, bacterial toxin effects, inflammatory mediators, etc., and involving membrane rupture and release of intracellular contents to the surrounding tissue, often with harmful inflammatory consequences. Necrotic cells may be detected and characterized by detection of compromised cell membranes, e.g. by methods such as staining with propidium iodide followed by flow cytometry or microscopy.

United States Patent Number 5,908,954 to Bolton, issued November 9, 1999, discloses an autoimmune vaccine for treatment of autoimmune diseases, prepared by subjecting autologous blood *ex vivo* to stressors, namely oxidizing agents, UV radiation and/or elevated temperatures. The vaccine so produced is reported to contain lymphocytes and other leukocytes exhibiting a condensed apoptotic-like morphology, in greater numbers than are contained in normal blood of the patient. Such cells are different and distinct from apoptotic cells and apoptotic bodies. Repeats of the procedures described in this patent, namely subjection of whole human blood to an ozone/oxygen gas mixture containing 14 - 15 mcg/mL ozone/medical grade oxygen bubbled through the blood aliquot at a rate of about 200mLs per minute for three minutes at a temperature of 42.5°C whilst subjected to UV radiation of wavelength 253.7 nm, produced a white blood cell fraction which, when separated and analyzed by Annexin 5 staining using "Annex 5 FTIC Apoptosis Detection Kit" (Sigma Product Cord/APO/AF) showed virtually no presence of apoptotic cells or apoptotic bodies.

### Summary of the Invention

The object of the invention is defined in the claims.

According to the present invention, the administration of apoptotic cells and/or apoptotic bodies previously prepared *ex vivo,* can be used in the prophylaxis and/or treatment of inflammatory and T-cell-mediated medical disorders. The medical disorder can be a T-cell-mediated disease such as rheumatoid arthritis, scleroderma, lupus, diabetes, psoriasis etc. It can be an inflammatory disorder resulting from an allergic reaction of the body or an organ thereof to an externally contacted or ingested substance, for example, contact hypersensitivity, asthma, poison ivy or poison oak reaction, nettle rash, etc., and infections. It can also be inflammatory, allergic or T-cell-mediated disorders of internal organs such as liver disorders, kidney disorders, heart disorders, and the like.

This invention can be used for the treatment of or prophylaxis against T -cell-mediated and inflammatory disorders in a mammalian patient, said treatment comprising administering to the patient an effective amount of apoptotic bodies and/or apoptotic cells.

Treatment is preferably accomplished by administering to the patient the pharmaceutical compositions described herein.

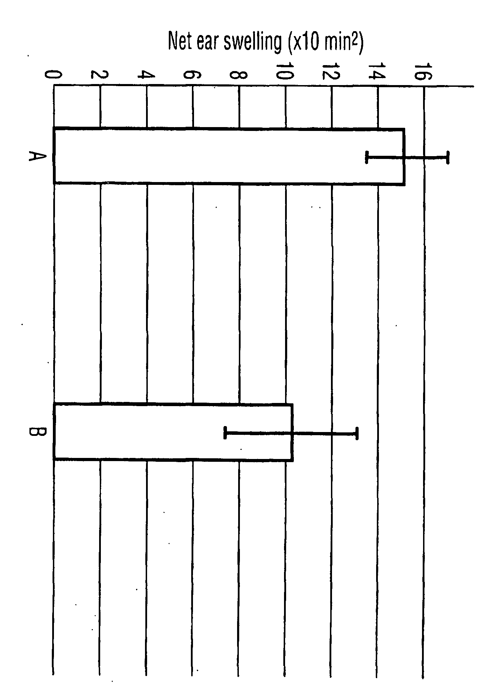

### Brief Description of the Drawing

The Figure is a graph showing a comparison of net ear swelling in mice treated with the compositions of this invention and a control group.

### Description of the Preferred Embodiments

This invention is directed to the use of apoptotic cells and/or apoptotic bodies for the treatment of T-cell-mediated and inflammatory disorders in mammalian patients.

"Apoptotic cells" and "apoptotic bodies," as the terms are used herein, means cells and cell bodies which exhibit one or more of the following apoptosis-characterizing features: surface exposure of phosphatidylserine, as detected by standard, accepted methods of detection such as Annexin V staining; alterations in mitochondrial membrane permeability measured by standard, accepted methods (e.g. Salvioli, S., Ardizzoni, A., Franceschi, C. Cossarizza, A. (1997) "JC-1, but not DiOC6(3) or Rhodamine 123, is a Reliable Fluorescent Probe to assess Delta Psi Changes in Intact Cells: Implications for Studies on Mitochondrial Functionality during Apoptosis," *FEBS Letters* **411:** 77-82]; evidence of DNA fragmentation such as the appearance of DNA laddering on agarose gel electrophoresis following extraction of DNA from the cells [Teiger, E., Dam, T.V., Richard, L., Wisnewsky, C., Tea, B.S., Gaboury, L., Tremblay, J., Schwartz, K. and Hamet, P. (1996) "Apoptosis in Pressure Overload-induced Heart Hypertrophy in the Rat," *Journal of Clinical Investigation* **97;** 2891-2897], or by *in situ* labeling (see Gavrieli et al., 1992, referenced above).

The apoptotic cells and/or apoptotic bodies for use in the present invention preferably comprise not more than about 35 weight percent of necrotic cells and/or necrotic bodies based on the total weight of the apoptotic cells/bodies and necrotic cells/bodies; more preferably, not more than about 20 weight percent; and even more preferably, not more than about 10 weight percent. At these levels, the presence of such necrotic cells and/or bodies is believed not to significantly alter *in vivo* processes. In its most preferred embodiment, the apoptotic cells/bodies are substantially free of necrotic cells and/or bodies (i.e., less than about 2 weight percent of necrotic cells/bodies).

The apoptotic cells and/or apoptotic bodies for use in the present invention are prepared *ex vivo* from mammalian cells that are compatible with those of the mammalian patient. They can be prepared from substantially any type of mammalian cell including cultured cell lines. Preferably they are prepared from a cell type derived from the mammalian patient's own body or from an established cell line. More preferably they are prepared from white blood cells of blood compatible with that of the mammalian patient, more preferably from the patient's own white blood cell and even more preferably from the patient's own T lymphocytes. Even more preferably they are prepared from an established cell line. The apoptotic cells and/or apoptotic bodies are prepared extracorporeally prior to administration to the patient. Thus, in one embodiment, an aliquot of the patient's blood may be withdrawn, e.g. by venipuncture, and at least a portion of the white cells thereof subjected extracorporeally to apoptosis inducing conditions.

A variety of methods of inducing apoptosis in mammalian cells, so as to create apoptotic cells and apoptotic bodies, are known in the art and essentially any of these can be adopted in preparing apoptotic bodies for use in the present invention. One such method is the subjection of the cells to ionizing radiation (y-rays, x-rays, etc.) and/or non ionizing electromagnetic radiation including ultraviolet light. Apoptosis can also be induced by subjecting cells to ultrasound.

Another method is the treatment of the cells with drugs such as non-specific protein kinase inhibitors as exemplified by staurosporine (see Bombeli, Karsan, Tait and Hirlan, (1997) "Apoptotic Vascular Endothelial Cells Become Procoagulant", Blood, Vol. 89:2429-2442). Also, certain chemotherapeutic agents used for the treatment of malignant tumours induce apoptosis, for example adriamycin, as can statin drugs (3-hydroxy-3methylglutaryl coenzyme A reductase inhibitors) [Guijarro C, Blanco-Colio LM, Ortego M, Alonso C, Ortiz A, Plaza JJ, Diaz C, Hernandez G, Edigo J (1998), "3-hydroxy-3methylglutaryl coenzyme A reductase and isoprenylation inhibitors induce apoptosis of vascular smooth muscle in culture," *Circulation Research* **83:** 490-500] and colcicine [Suzuki Y (1998)", "Cell death, phagocytosis and neurogenesis in mouse olfactory epithelium and vomeronasal organ after colcicine treatment," *Annals of the New York Academy of Sciences* **855:** 252-254]. The use of ligands for death receptors on cells, such as Fas-ligand, will be apparent for inducing apoptosis from the discussion of apoptosis above.

Yet another method is the application of oxidative stress to cells extracorporeally (see for example Buttke and Sandstrom (1994) "Oxidative Stress as a Mediator of Apoptosis," *Immunology Today,* 15:7-10). This can be achieved by treating the cells, in suspension, with chemical oxidizing agents such as hydrogen peroxide, other peroxides and hydroperoxides, ozone, permanganates, periodates, and the like. Biologically acceptable such oxidizing agents are preferably used, so as to reduce potential problems associated with residues and contaminations of the apoptotic cells and apoptotic bodies so formed.

The present invention is not restricted to any particular method of producing apoptotic cells and apoptotic bodies for use herein, and any suitable, known process can be used.

Methods for the detection and quantitation of apoptosis can be used to determine the presence and level of apoptosis in the preparation to be administered to the patient in the present invention. At least one of the methods from those described in the introduction above should be used to confirm the level of apoptosis achieved prior to administration. They are suitably purified prior to use, by methods known in the art, such as differential centrifugation.

In preparing the apoptotic cells and/or apoptotic bodies, care should be taken not to apply excessive levels of oxidative stress, radiation, drug treatment, etc., since otherwise there is a significant risk of causing necrosis of at least some of the cells under treatment. Necrosis causes cell membrane rupture and the release of cellular contents often with biologically harmful results, particularly inflammatory events, so that the presence of necrotic cells and their components along with the apoptotic bodies is best avoided.

Appropriate levels of treatment of the cells to create apoptotic bodies for use in the present invention depend to some extent on the nature of the chosen cells and cellular composition, and the type of treatment chosen to induce apoptosis. Such appropriate levels are readily determinable by those skilled in the art, having regard to the available scientific literature on the subject including the above-referenced articles.

One preferred process according to the present invention involves the culture of cells from the patient, or a compatible mammalian cell line. The cultured cells may then be treated to induce apoptosis and to create apoptotic cells and/or apoptotic bodies therein. The cells, suspended in the patient's plasma or another suitable suspension medium, such as saline or a balanced mammalian cell culture medium, can then be administered as indicated below. The numbers of apoptotic cells and/or bodies can be determined by published methods available in the scientific literature on the subject including the above-referenced articles. The numbers of such apoptotic cells and/or apoptotic bodies required for administration to the patient to obtain the required clinical benefit will vary depending on the source of cells, the patient's condition, the age and weight of the patient and other relevant factors which are readily determinable by the attending clinician.

Thus an example of a preferred process according to the present invention accordingly involve taking an extracted aliquot of blood from the patient to be treated, and treatment of the white cells thereof under apoptosis-causing conditions, so as to create a cellular composition in which significant numbers of the white cells therein have been apoptosed so as to create therein substantial numbers of apoptotic cells or bodies. The treated composition is then to be re-administered to the patient.

The aliquot treated to cause apoptosis may be whole blood, but is preferably a separated white cell fraction thereof, separated from the blood by known means, and suspended in plasma or another suitable suspension medium, such as saline or a balanced mammalian cell culture medium. More preferably, T lymphocytes, isolated from the blood by known means, and suspended as above, may be used as a source of apoptotic cells and apoptotic bodies.

The number of viable cells selected for treatment to create apoptotic cells and/or apoptotic bodies is suitably up to about 4 x 10⁹, preferably from about 1,000,000 to about 1,000,000,000 and most preferably from about 50,000,000 to about 150,000,000, for each administration to a human patient. From about 10% to 90%, preferably from about 30% to 70% of the cellular composition for administration is comprised of apoptotic cells and apoptotic bodies, the balance being viable cell and necrotic cells. Accordingly, the preferred amounts of apoptotic cells and/or apoptotic bodies for administration are those produced by subjecting these numbers of cells to the apoptosing conditions. When whole blood is used as the source of the cells to be subjected to the apoptosis inducing conditions, these numbers of white cells are obtainable in blood aliquots of volume up to about 400 ml, preferably up to 100 ml. More specifically, 50,000,000 to 150,000,000 cells is equivalent to the white cells in blood aliquots of volume 10 - 30 ml.

The volume of the aliquot of blood withdrawn from the patient for treatment to create apoptotic cells and/or apoptotic bodies therein is suitable up to about 400 ml, preferably from about 0.1 to about 100 ml, and most preferably from about 5 to about 15 ml. Accordingly, the preferred amounts of apoptotic cells and /or apoptotic bodies for administration are those corresponding to the numbers derivable from the white blood cells, or isolated T lymphocytes, contained in such quantities of whole blood, following subjection to apoptosis-inducing conditions.

The suspension of treated apoptotic cells and/or bodies for administration to the patient is prepared in a biologically acceptable liquid suspending medium, such as the patient's serum or plasma, saline or balanced mammalian cell culture medium. The addition of other factors, such as cytokines, hormones, products of stressed cells or other appropriate biologically active material may enhance the benefit of the administered apoptotic cellular materials. The aliquot can be re-introduced into the patient's body by any suitable method, most preferably intramuscular injection but also including subcutaneous injection, mini-grafting, intra-peritoneal injection, intra-arterial injection, intravenous injection and oral administration. The apoptotic entities can be delivered to the specific body organ and/or site by using any appropriate, known delivery system.

The compositions of this invention may optionally include a pharmaceutically acceptable excipient. Some examples of suitable excipients include sterile water, sterile saline, phosphate buffered saline, and the like.

When administered, the pharmaceutical compositions comprise an effective amount of apoptotic bodies/cells to induce a suitable prophylactic and/or therapeutic response in the patient at risk of suffering or suffering from a T-cell mediated or inflammatory disease. Preferably, the composition administered to the mammalian patient comprises from about 10,000 to 10,000,000 apoptotic cells or bodies per kilogram of body weight, more preferably from about 500,000 to 5,000,000 and most preferably from about 1,500,000 to 4,000,000 apoptotic cells or bodies per kg body weight. The specific dose employed will, of course, be dependent upon the age, weight and severity of the disease in the treated patient all of which are within the skill of the attending clinician.

For most effective treatment and/or prophylaxis of mammalian disorders involving an inflammatory component, the patient may be given a course of treatments with apoptotic cells and/or bodies according to the invention. Each course of treatment may involve administration to the patient of from 1 to 6 aliquots of suspended cellular material, as described above. No more than one such aliquot should be administered per day, and the maximum rest period between any two consecutive administrations should be not greater than about 21 days. Booster treatments as described below may advantageously be used. To maintain the desired effects, the patient may undergo booster treatments, with a further course of administration of aliquots of suspended apoptotic cells and/or apoptotic bodies as described above, at intervals of three to four months.

As noted, the present invention is applicable to the treatment and/or prophylaxis of a wide variety of mammalian T-cell-mediated and inflammatory disorders such as autoimmune disorders. These include lupus, diabetes, scleroderma, psoriasis and rheumatoid arthritis, as well as inflammatory allergic reactions, organ and cell transplantation reaction disorders, and microbial infections giving rise to inflammatory reactions. The present invention is also applicable to preconditioning against ingestion of poisons, exposure to toxic chemicals, radiation damage, and exposure to airborne and water borne irritant substances, etc., which cause damaging inflammation. In addition, it is applicable to inflammatory, allergic and T-cell-mediated disorders of internal organs such as kidney, liver, heart, etc.

The invention is further described, for illustrative purposes, in the following specific examples.

### EXAMPLE 1

Experiments to demonstrate the invention were conducted on laboratory mice, under approved conditions for conducting such experiments.

The effectiveness of the treatment according to a preferred embodiment of the present invention, on contact hypersensitivity (CHS), an example of a Th-1-cell inflammatory disorder which is known to be mediated by inflammatory cytokines, was assessed on laboratory mice, according to approved animal experimentation procedures, using the method described by Kondo et. al., "Lymphocyte function associated antigen-1 (LFA-1) is required for maximum elicitation of allergic contact dematitis" Br. J. Dermatol. **131**:354-359 (1994), with minor variations. The disclosure thereof is incorporated herein by reference. Briefly, to induce CHS, the abdominal skin of each mouse was shaved and painted with dinitrodifluorobenzene DNFB, the sensitizing chemical, using 25 µl of 0.5% DNFB in 4:1 acetone:olive oil solution. This sensitization was applied to two groups of Balb/c mice, 10 animals in total.

Apoptotic bodies were prepared from murine fibroblasts. The murine fibroblasts were treated with 50 mM sodium butyrate in RPMI medium, at confluency for one day, and then the sodium butyrate medium was changed. To increase the number of apoptotic cells and bodies, the cells can additionally be irradiated with UV-light (e.g. 75 mj). Supernatant containing floating cells is removed 24 hours following irradiation.

Apoptotic bodies were quantitated by centrifuging the supernatant (1200 rpm, 5 minutes), aspirating the supernatant, washing the resulting cell pellet with PBS and centrifuging again, as above. The pellet containing the apoptotic bodies was re-suspended in PBS. The cells were stored in PBS at 4°C for the duration of the experiment. The cells to be stained for quantitation were re-suspended in 1X binding buffer at a concentration of 1x10⁶ cells/ml. 100 µl of the cells were transferred to a 5 ml tube, and 10 µl of fluorescein-conjugated annexin V and 10 µl propidium iodide reagent was added. The cells were gently vortexed and the cell mixture incubated for 15 minutes at 25°C in the dark. Following the incubation, 400 µl of 1X binding buffer was added to each tube. The sample was analyzed on a flow cytometer over one hour.

Of the two groups of sensitized mice, the first, control group A, received no treatment. The second, test group B, was treated with an injection of suspended apoptotic bodies prepared as described above, 50 µl volume containing at least 150,000 bodies per injection of blood subjected to stressors as described above. Treatments, each involving intramuscular injection of 50 µl of the respective liquid, started on the day of sensitization, and were repeated every day for a total of six days. On the same day as the last treatment, but after its administration, the animals were challenged with DNFB, by applying to the right ear of each animal 10 µl of 0.2% solution of DNFB in acetone and olive oil. To the left ear of each animal was applied the acetone/olive oil solvent, without DNFB. Inflammation due to CHS manifests itself in a swelling of the right ears. Ear thickness was measured, 24 hours after challenge, with a Peacock spring-loaded micrometer (Ozaki Co., Tokyo, Japan). The results were expressed as the thickness and difference in thickness of the right ears and the left ears of each animal, at 24 hours after challenge.

The experiments were repeated, using more sets of two groups of animals, a sufficient number of times to ensure statistical significance in the results. A notable and significant reduction in ear thickness (inflammation) was observed with the animals treated with the apoptotic cells and apoptotic bodies suspension in accordance with the invention, as compared with the untreated group, demonstrating a significant reduction in inflammation. The results are presented in the following Table, and on the accompanying Figure, as a bar graph of net ear swelling (difference between right ear and left ear thickness), for each group, with "standard deviation" shown by the vertical line at the top of each column.

**TABLE 1**

| Group | Leftear | Rightear | Difference |
|---|---|---|---|
| A | 17 | 31 | 14 |
| A | 18 | 39 | 21 |
| A | 17 | 30 | 13 |
| A | 18 | 32 | 14 |
| A | 18 | 31 | 13 |
| | | | Mean: 15 S.D: 3.391165 |

| Group | Leftear | Rightear | Difference |
|---|---|---|---|
| B | 21 | 31 | 10 |
| B | 18 | 18 | 0 |
| B | 17 | 30 | 13 |
| B | 20 | 24 | 4 |
| B | 18 | 22 | 4 |
| | | | Mean: 6.2 S.D.: 5.215362 |

An analysis of the suspension of apoptotic cells and bodies administered to the animals of test group B indicated the presence therein of approximately 40% apoptotic cells and bodies, balance viable cells and minor amounts of necrotic cells (not more than 20%), the presence of which is believed not to be significant in the *in vivo* process.

### EXAMPLE 2

The above test procedure was repeated on similar groups of animals, a control group and a test group, but using a suspension of apoptotic cells and bodies on the test group which comprised about 60% apoptotic cells and bodies, balance viable cells and a minor amount (not more than 20%) of necrotic cells. Essentially similar results were obtained.

The effectiveness of the processes and compositions of the present invention in preventing and alleviating inflammation due to CHS indicates that administration of apoptotic cells and bodies as described up-regulates the *in vivo* generation of anti-inflammatory Th-2 derived cytokines such as IL-10 (known to be implicated in CHS, see Kondo, McKenzie and Sauder, "The Journal of Investigative Dermatology," Vol. 103, 1994, page 811-814) and, perhaps as a consequence, down-regulates inflammatory cytokines such as TNFy, IL-6 and IL-12. These inflammatory cytokines are implicated in autoimmune diseases such as psoriasis, rheumatoid arthritis, scleroderma, lupus, diabetes mellitus, organ rejection, miscarriage, multiple sclerosis, inflammatory bowel disease and atherosclerosis, as well as graft versus host disease. Consequently, the finding of success in CHS treatment reported in the above Examples is indicative of successful use of the process and compositions in the treatment and/or prophylaxis of a wide variety of T-cell-mediated and inflammatory disorders including those discussed above.

## Claims

1. The use of a cellular composition comprising apoptotic bodies and/or apoptotic cells in the preparation of a medicament for the treatment and/or prophylaxis of a disorder selected from T-cell mediated and inflammatory disorders, in a mammalian patient, the apoptotic bodies and/or apoptotic cells being compatible with the blood cells of the patient.

2. Use as in claim 1 wherein the medicament is a liquid suspension.

3. Use according to claim 1 or claim 2 wherein the apoptotic bodies and/or apoptotic cells are derived from extracorporeal treatment of blood cells compatible with those of the mammalian patient.

4. Use according to claim 3 wherein the blood cells are white cells.

5. Use according to claim 4 wherein the white blood cells are the patient's own white blood cells.

6. Use according to claim 5 wherein the blood cells are the patient's own T-lymphocytes.

7. Use according to claim 1 or claim 2 wherein the apoptotic bodies and/or cells are derived from established cultured cell lines.

8. Use according to any preceding claim wherein the cellular composition comprises not more than 20 weight per cent of necrotic cells/bodies based upon the total weight of the apoptotic cells/bodies and necrotic cells/bodies.

9. Use according to claim 8 wherein the cellular composition in substantially free of necrotic cells and/or bodies.

10. Use according to any preceding claim wherein the disorder is selected from the group consisting of psoriasis, rheumatoid arthritis, scleroderma, lupus, diabetes mellitus, organ rejection, miscarriage, multiple sclerosis, inflammatory bowel disease, atherosclerosis, and graft versus host disease.

11. Use according to any of claims 1 - 9 wherein the disorder is contact hypersensitivity.

12. Use according to any preceding claim in preparation of a unit dosage of medicament for administration to a human patient, said dosage containing from 10,000 to 10,000,000 apoptotic bodies and/or apoptotic cells per kilogram body weight of the patient.

13. Use according to claim 12 wherein said dosage contains from 500,000 to 5,000,000 apoptotic bodies and/or apoptotic cells per kilogram body weight of the patient.

14. Use according to claim 13 wherein said dosage contains from 1,500,000 to 4,000,000 apoptotic bodies and/or apoptotic cells per kilogram body weight of the patient.

15. Use according to any preceding claim wherein the apoptotic bodies and/or apoptotic cells constitute from 10% to 90% of the cellular portion of the medicament

16. Use according to claim 14 wherein the apoptotic bodies and/or apoptotic cells constitute from 30% to 70% of the cellular portion of the medicament.

17. A pharmaceutical composition comprising a liquid suspension of mammalian cellular material including apoptotic bodies and/or apoptotic cells, said mammalian cellular material comprising not more than 20 weight per cent of necrotic cells/bodies based on the total weight of the apoptotic cells/bodies and necrotic cells/bodies.

18. The composition of claim 17 which is substantially free of necrotic cells and/or bodies.

19. The composition of claim 17 or claim 18 wherein from 10% to 90% of the cellular material is apoptotic bodies and/or apoptotic cells.

20. The composition of claim 19 wherein from 30% to 70% of the cellular material is apoptotic bodies and/or apoptotic cells.

## Patentansprüche

1. Verwendung einer zellulären Zusammensetzung umfassend apoptotische Körper und/oder apoptotische Zellen in der Herstellung eines Medikaments für die Behandlung und/oder Prophylaxe einer Erkrankung, die ausgewählt ist aus T-Zellvermittelten und Entzündungserkrankungen, bei einem Säugetierpatienten, wobei die apoptotischen Körper und/oder apoptotischen Zellen mit den Blutzellen des Patienten verträglich sind.

2. Verwendung nach Anspruch 1, wobei das Medikament eine flüssige Suspension ist

3. Verwendung nach Anspruch 1 oder Anspruch 2, wobei die apoptotischen Körper und/oder apoptotischen Zellen aus einer extrakorporalen Behandlung von Blutzellen erhalten werden, die mit denen des Säugetierpatienten verträglich sind.

4. Verwendung nach Anspruch 3, wobei die Blutzellen Leukozyten sind.

5. Verwendung nach Anspruch 4, wobei die Leukozyten die eigenen Leukozyten des Patienten sind.

6. Verwendung nach Anspruch 5, wobei die Blutzellen die eigenen T-Lymphozyten des Patienten sind.

7. Verwendung nach Anspruch 1 oder Anspruch 2, wobei die apoptotischen Körper und/oder Zellen aus etablierten gezüchteten Zelllinien erhalten werden.

8. Verwendung nach einem der vorangehenden Ansprüche, wobei die zelluläre. Zusammensetzung nicht mehr als 20 Gew.% nekrotische Zellen/Körper basierend auf dem Gesamtgewicht der apoptotischen Zellen/Körper und nekrotischen Zellen/Körper umfaßt.

9. Verwendung nach Anspruch 8, wobei die zelluläre Zusammensetzung im wesentlichen frei von nekrotischen Zellen und/oder Körpern ist.

10. Verwendung nach einem der vorangehenden Ansprüche, wobei die Erkrankung ausgewählt wird aus der Gruppe bestehend aus Psoriasis, rheumatischer Arthritis, Skleroderma, Lupus, Diabetes mellitus, Organabstoßung, Fehlgeburt, Multiple Sklerose, entzündliche Dannerkrankung, Atherosklerose und Graft-versus-Host-Krankheit.

11. Verwendung nach einem der Ansprüche 1 bis 9, wobei die Erkrankung Kontakthypersensibilität ist.

12. Verwendung nach einem der vorangehenden Ansprüche in Bereitung einer Einheitsdosierung eines Medikaments zur Verabreichung an einen menschlichen Patienten, wobei die Dosierung 10.000 bis 10.000.000 apoptotische Körper und/oder apoptotische Zellen pro Kilogramm Körpergewicht des Patienten enthält.

13. Verwendung nach Anspruch 12, wobei die Dosierung 500.000 bis 5.000.000 apoptotische Körper und/oder apoptotische Zellen pro Kilogramm Körpergewicht des Patienten enthält.

14. Verwendung nach Anspruch 13, wobei die Dosierung 1.500.000 bis 4.000.000 apoptotische Körper und/oder apoptotische Zellen pro Kilogramm Körpergewicht des Patienten enthält.

15. Verwendung nach einem der vorangehenden Ansprüche, wobei die apoptotischen Körper und/oder apoptotischen Zellen 10% bis 90% des zellulären Anteils des Medikaments ausmachen.

16. Verwendung nach Anspruch 14, wobei die apoptotischen Körper und/oder apoptotischen Zellen 30% bis 70% des zellulären Anteils des Medikaments ausmachen.

17. Pharmazeutische Zusammensetzung umfassend eine flüssige Suspension eines zellulären Säugetiermaterials einschließend apoptotische Körper und/oder apoptotische Zellen, wobei das zelluläre Säugetiermaterial nicht mehr als 20 Gew,% an nekrotischen Zellen/Körpern basierend auf dem Gesamtgewicht der apoptotischen Zellen/Körper und nekrotischen Zellen/Körper umfaßt.

18. Zusammensetzung nach Anspruch 17, die im wesentlichen frei von nekrotischen Zellen und/oder Körpern ist.

19. Zusammensetzung nach Anspruch 17 oder Anspruch 18, wobei 10% bis 90% des zellulären Materials apoptotische Körper und/oder apoptotische Zellen sind.

20. Zusammensetzung nach Anspruch 19, wobei 30% bis 70% des zellulären Materials apoptotische Körper und/oder apoptotische Zellen sind.

## Revendications

1. Utilisation d'une composition cellulaire comprenant des corps apoptotiques et/ou des cellules apoptotiques dans la préparation d'un médicament pour le traitement et/ou la prophylaxie d'un trouble choisi parmi des troubles médiés par les cellules T et inflammatoires, chez un patient mammifère, les corps apoptotiques et/ou cellules apoptotiques étant compatibles avec les cellules sanguines du patient.

2. Utilisation selon la revendication 1, dans laquelle le médicament est une suspension liquide.

3. Utilisation selon la revendication 1 ou la revendication 2, dans laquelle les corps apoptotiques et/ou cellules apoptotiques sont dérivé(e)s d'un traitement extracorporel de cellules sanguines compatibles avec celles du patient mammifère.

4. Utilisation selon la revendication 3, dans laquelle les cellules sanguines sont des leucocytes.

5. Utilisation selon la revendication 4, dans laquelle les leucocytes sont les propres leucocytes du patient.

6. Utilisation selon la revendication 5, dans laquelle les leucocytes sont les propres lymphocytes T du patient.

7. Utilisation selon la revendication 1 ou la revendication 2, dans laquelle les corps et/ou cellules apoptotiques sont dérivé(e)s de lignées cellulaires établies cultivées.

8. Utilisation selon l'une quelconque des revendications précédentes, dans laquelle la composition cellulaire ne comprend pas plus de 20 pour cent en poids de cellules/corps nécrotiques sur la base du poids total des cellules/corps apoptotiques et des cellules/corps nécrotiques.

9. Utilisation selon la revendication 8, dans laquelle la composition cellulaire est essentiellement exempte de cellules et/ou corps nécrotiques.

10. Utilisation selon l'une quelconque des revendications précédentes, dans laquelle le trouble est choisi parmi le groupe comprenant le psoriasis, la polyarthrite rhumatoïde, la sclérodermie, le lupus, le diabète sucré, le rejet d'organes, l'avortement spontané, la sclérose en plaques, la maladie inflammatoire des intestins, l'athérosclérose et la maladie du greffon contre l'hôte.

11. Utilisation selon l'une quelconque des revendications 1 à 9, dans laquelle le trouble est une hypersensibilité de contact.

12. Utilisation selon l'une quelconque des revendications précédentes dans la préparation d'une dose unitaire de médicament destinée à être administrée à un patient humain, ladite dose contenant de 10 000 à 10 000 000 de corps apoptotiques et/ou cellules apoptotiques par kilogramme de poids corporel du patient.

13. Utilisation selon la revendication 12, dans laquelle ladite dose contient de 500 000 à 5 000 000 de corps apoptotiques et/ou cellules apoptotiques par kilogramme de poids corporel du patient.

14. Utilisation selon la revendication 13, dans laquelle ladite dose contient de 1 500 000 à 4 000 000 de corps apoptotiques et/ou cellules apoptotiques par kilogramme de poids corporel du patient.

15. Utilisation selon l'une quelconque des revendications précédentes, dans laquelle les corps apoptotiques et/ou cellules apoptotiques constituent de 10 % à 90 % de la fraction cellulaire du médicament.

16. Utilisation selon la revendication 14, dans laquelle les corps apoptotiques et/ou cellules apoptotiques constituent de 30 % à 70 % de la fraction cellulaire du médicament.

17. Composition pharmaceutique comprenant une suspension liquide de matériel cellulaire de mammifère incluant des corps apoptotiques et/ou cellules apoptotiques, ledit matériel cellulaire de mammifère ne comprenant pas plus de 20 pour cent en poids de cellules/corps nécrotiques sur la base du poids total des cellules/corps apoptotiques et des cellules/corps nécrotiques.

18. Composition selon la revendication 17, laquelle est essentiellement exempte de cellules et/ou corps nécrotiques.

19. Composition selon la revendication 17 ou 18, dans laquelle de 10 % à 90 % du matériel cellulaire est des corps apoptotiques et/ou cellules apoptotiques.

20. Composition selon la revendication 19, dans laquelle de 30 % à 70 % du matériel cellulaire est des corps apoptotiques et/ou cellules apoptotiques.
